# EUROPEAN PATENT APPLICATION

(11) **EP 4 641 206 A1**
(43) Date of publication of application: **29.10.2025**
(21) Application number: 22969098.7
(22) Date of filing: 19.12.2022
(51) Int. Cl.: G01N 35/00

(54) **ANALYSIS DEVICE AND ULTRAVIOLET IRRADIATION UNIT**

(71) Applicant: HITACHI HIGH-TECH CORPORATION, Tokyo 105-6409 (JP)
(72) Inventor: MAKINO, Yoko, Tokyo 105-6409 (JP); SHIBAHARA, Masashi, Tokyo 105-6409 (JP); SATOH, Wataru, Tokyo 100-8280 (JP); YAMAGATA, Toshiki, Tokyo 105-6409 (JP)
(74) Representative: Strehl & Partner mbB
(86) International application number: PCT/JP2022/046602
(87) International publication number: WO 2024/134708

(57) **Abstract**

The present invention comprises a detection part 6 that performs analysis of a sample, a sample installation part 2 that performs necessary processing in analysis by the detection part 6, a disposal box 3, a reaction part 5, an unused consumables area 7, an operating part 8, a reagent storage 9, an extraction part 10, and a stage 1 that constitutes a portion of a housing of an analysis device 30, and the present invention is provided with an ultraviolet source 14 that radiates ultraviolet light to a target position on the stage 1, and an x-axis-direction actuator 16, a y-axis-direction actuator 17, and a z-axis-direction actuator 18 that move the ultraviolet source 14 in an x-direction as a first direction of the stage 1, a y-direction at a right angle to the x-direction, and a z-direction as the vertical direction, respectively. An analysis device and ultraviolet irradiation unit in which it is possible to radiate ultraviolet light to only a prescribed range are thereby provided.

## Description

### Technical Field

The present invention relates to an analysis device and an ultraviolet irradiation unit.

### Background Art

As an example of an ultraviolet sterilization device that uniformly irradiates an outer surface of a target to be sterilized with ultraviolet light, PTL 1 discloses the following configuration.

"A sterilization chamber, an ultraviolet irradiation part, and a placement table are provided. The ultraviolet irradiation part is provided in the sterilization chamber to emit ultraviolet light to a target to be sterilized. The placement table is provided in the sterilization chamber, and the target to be sterilized is placed thereon. The placement table is formed of a material that transmits ultraviolet light. The ultraviolet irradiation part surrounds the placement table".

### Citation List

### Patent Literature

PTL 1: JP6032934B

### Summary of Invention

### Technical Problem

In an automatic analyzer that analyzes a sample such as blood or urine, there may be a demand for ultraviolet irradiation in order to sterilize a stage or a consumable and to reduce virus infectivity.

In an ultraviolet irradiation method inside a device in the related art, relative to a portion to be irradiated, an ultraviolet irradiation part is mounted at one location or surrounds the inside of the device to enable uniform ultraviolet irradiation (for example, see PTL 1).

In such a method in the related art as disclosed in PTL 1 or the like, when there is a portion where ultraviolet irradiation is desired to be avoided, it is required to remove a light source corresponding to the portion or provide a cover to prevent ultraviolet light from reaching the portion.

However, such a countermeasure as described above is not an operation that can be easily performed, and there is a demand for a mechanism that performs irradiation while avoiding the portion where ultraviolet irradiation is desired to be avoided.

An object of the invention is to provide an analysis device and an ultraviolet irradiation unit that can emit ultraviolet light only to a predetermined range.

### Solution to Problem

The invention includes a plurality of aspects for solving the above problem, and an example thereof is an analysis device including: an analysis part configured to perform analysis of a sample; a processing part configured to perform necessary processing in analysis performed by the analysis part; a stage on which the analysis part and the processing part are disposed and which constitutes a portion of a housing of the analysis device; an ultraviolet source configured to emit ultraviolet light to a target position on the stage; and a conveyance mechanism configured to move the ultraviolet source in an x-direction that is one direction of the stage, a y-direction at a right angle to the x-direction, and a z-direction that is a vertical direction.

### Advantageous Effects of Invention

According to the invention, it is possible to emit ultraviolet light only to a predetermined range. Problems, configurations, and effects other than those described above will be clarified by the following description of embodiments.

### Brief Description of Drawings

[FIG. 1] FIG. 1 is a diagram showing a schematic configuration of an analysis device according to a first embodiment.
[FIG. 2] FIG. 2 is a diagram showing a schematic configuration of an ultraviolet irradiation unit according to the first embodiment and an overview of a moving method therefor.
[FIG. 3] FIG. 3 is a diagram showing a schematic configuration of an analysis device according to a second embodiment.
[FIG. 4] FIG. 4 is a diagram showing a schematic configuration around an ultraviolet source in an ultraviolet irradiation unit according to the second embodiment.
[FIG. 5] FIG. 5 is a diagram showing an overview of a moving method for the ultraviolet irradiation unit according to the second embodiment.
[FIG. 6] FIG. 6 is a diagram showing the overview of the moving method for the ultraviolet irradiation unit according to the second embodiment.
[FIG. 7] FIG. 7 is a diagram showing a schematic configuration of an analysis device according to a third embodiment.
[FIG. 8] FIG. 8 is a diagram showing a schematic configuration of an ultraviolet irradiation unit according to the third embodiment.
[FIG. 9] FIG. 9 is a diagram showing an overview of a moving method for the ultraviolet irradiation unit according to the third embodiment.
[FIG. 10] FIG. 10 is a diagram showing a schematic configuration of an ultraviolet irradiation unit according to a fourth embodiment.
[FIG. 11] FIG. 11 is a top view showing a positional relationship between a light guide and a dispensing tip in the ultraviolet irradiation unit according to the fourth embodiment.
[FIG. 12] FIG. 12 is a diagram showing a schematic configuration of an ultraviolet irradiation unit according to a fifth embodiment.
[FIG. 13] FIG. 13 is a diagram showing an example of display of an operating part in an analysis device according to a sixth embodiment.

### Description of Embodiments

Hereinafter, embodiments of an analysis device and an ultraviolet irradiation unit of the invention will be described with reference to the drawings. In the drawings used in the present description, the same or corresponding components are denoted by the same or similar reference signs, and repeated description of these components may be omitted.

### <First Embodiment>

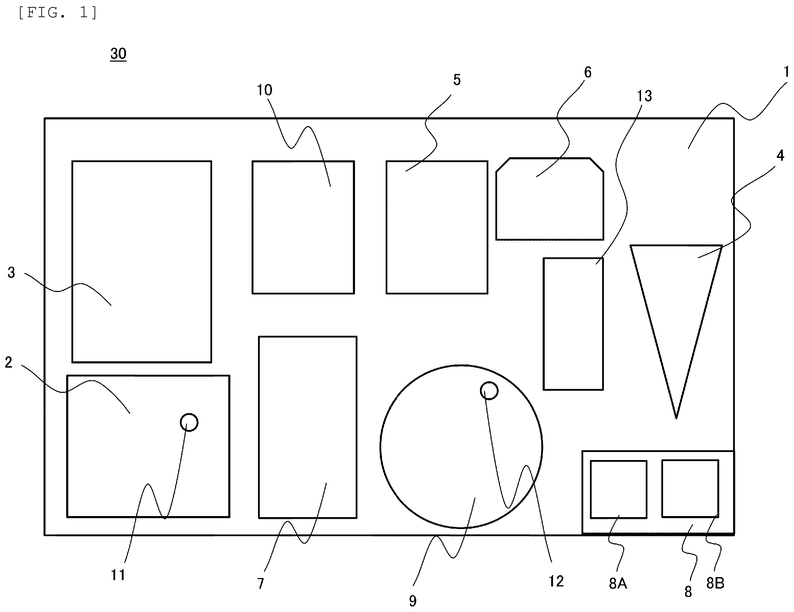

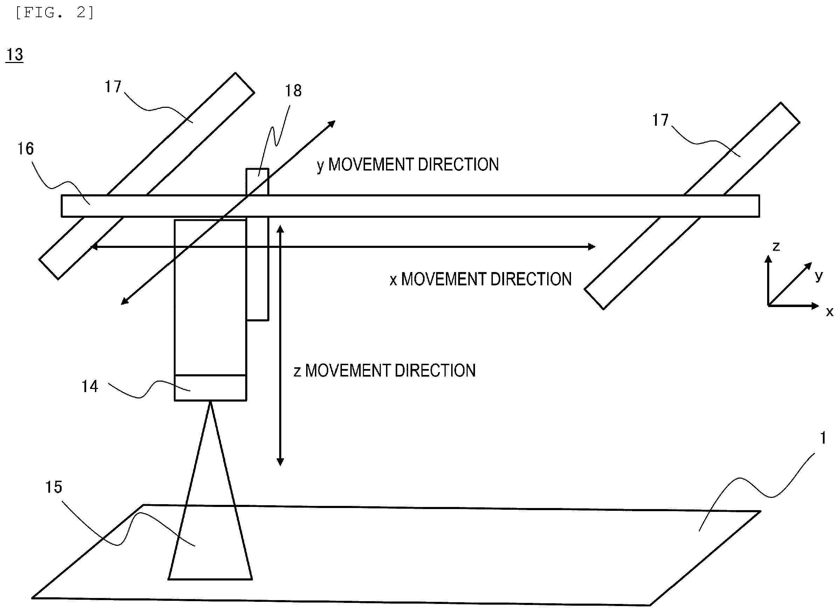

A first embodiment of the analysis device and the ultraviolet irradiation unit of the invention will be described with reference to FIGS. 1 and 2.

First, an overall configuration of the analysis device will be described with reference to FIG. 1. FIG. 1 is a schematic diagram of the analysis device of the first embodiment.

An analysis device 30 shown in FIG. 1 includes, on a stage 1 constituting a part of a housing of the analysis device 30, a sample installation part 2, a disposal box 3, an extraction part 10, a reaction part 5, a detection part 6, an unused consumables area 7, a reagent storage 9, an ultraviolet irradiation unit 13, an operating part 8, and the like.

The sample installation part 2 is a portion for loading and collecting a sample rack, and is a portion for temporarily storing one or more sample racks holding sample containers containing a loaded sample awaiting analysis and temporarily storing one or more sample racks before collection. The sample installation part 2 is provided with a sample dispensing hole 11.

The unused consumables area 7 is an area for placing and storing various consumables used for detecting the sample in the reaction part 5 and various consumables used for extracting a specific component from the sample in the extraction part 10.

The disposal box 3 is a box for disposing of used consumables and measured samples.

The reagent storage 9 is an area for placing and storing various reagents used for detecting the sample in the reaction part 5. The reagent storage 9 is provided with a reagent dispensing hole 12.

A dispenser 4 is a portion for dispensing the sample from the sample container placed on the sample rack on the sample installation part 2 to the extraction part 10 and for dispensing the reagent from the reagent storage 9 to biomolecules in the extraction part 10 or the reaction part 5. The dispenser 4 includes a drive mechanism in x-axis, y-axis, and z-axis directions and a dispensing probe, and moves above the sample installation part 2, the reagent storage 9, the extraction part 10, and the reaction part 5.

The extraction part 10 is a portion for purifying a test material containing a measurement target from the sample loaded in the sample installation part 2.

The reaction part 5 is a portion for reacting the test material purified by the extraction part 10 with the reagent.

The detection part 6 is a portion for measuring a product obtained in the reaction part 5.

Here, the numbers of the extraction part 10, the reaction part 5, and the detection part 6 are exemplary and may be any number of one or more. Similarly, an example in which there is one dispenser 4 is shown, and alternatively, two or more dispensers may be provided.

Further, each mechanism related to the stage 1 of the analysis device 30 is connected to the operating part 8 in a wired or wireless manner via a communication line.

An operation of the analysis device is controlled by the operating part 8. The operating part 8 includes a personal computer including a monitor 8A, a control part 8B, and the like.

The monitor 8A is a touch panel display that also serves as an input part, and displays various types of information on the analysis device 30, information on the sample to be analyzed, and information necessary for analysis. An input device such as a mouse or a keyboard may be separately provided as the input part.

The control part 8B is a portion that performs analysis computation processing based on a measurement result in the reaction part 5 and individually or entirely controls an operation of each part in the analysis device 30.

The control part 8B may be implemented as hardware by a dedicated circuit board, or may be implemented by software executed by a computer. When implemented by hardware, the control part 8B can be implemented by integrating a plurality of arithmetic units, which execute processing, on a wiring board or in a semiconductor chip or a package. When implemented by software, the control part 8B can be implemented by mounting a high-speed general-purpose CPU on a computer and executing a program for executing desired computation processing. It is also possible to upgrade an existing device by a recording medium in which the program is recorded. These devices, circuits, and computers are connected by a wired or wireless network, and data is transmitted and received as appropriate.

The above is the configuration of the analysis device 30 of the embodiment. The analysis device 30 does not need to include all mechanisms, and various mechanisms can be appropriately added and/or deleted as necessary.

Hereinafter, a flow of analysis in the analysis device 30 will be briefly described.

When the sample rack where the sample container is installed is set at the sample installation part 2 and analysis is started, the sample contained in the sample installation part 2 is dispensed by the dispenser 4 through the sample dispensing hole 11, and the aspirated sample is dispensed to the extraction part 10. Thereafter, the sample is purified and the test material is extracted by the extraction part 10 according to a requested item. The extracted test material is dispensed into the reaction part 5 by the dispenser 4. Thereafter, the test material is reacted with the reagent provided in the reagent storage 9, a predetermined reaction is performed in the reaction part 5, and measurement is performed in the detection part 6. A measurement result is recorded in a storage unit (not shown) of the operating part 8. After the measurement is completed, post-processing such as a step of disposing of the measured test material or the consumable in the disposal box 3 is performed, followed by transitioning to processing for measuring a next sample.

Next, a configuration and an operation of the characteristic ultraviolet irradiation unit according to the embodiment will be described with reference to FIG. 2. FIG. 2 is a diagram showing a schematic configuration of the ultraviolet irradiation unit according to the first embodiment and an overview of a moving method therefor.

The ultraviolet irradiation unit 13 shown in FIG. 2 includes an ultraviolet source 14 that irradiates a target position on the stage 1 with ultraviolet light, an x-axis-direction actuator 16 that moves the ultraviolet source 14 in an x-direction that is one direction of the stage 1, a y-axis-direction actuator 17 that moves the ultraviolet source 14 in a y-direction at a right angle to the x-direction, and a z-axis-direction actuator 18 that moves the ultraviolet source 14 in a z-direction that is a vertical direction relative to the x-direction, and the ultraviolet source 14 emits ultraviolet light 15.

Each of the x-axis-direction actuator 16, the y-axis-direction actuator 17, and the z-axis-direction actuator 18 includes, for example, two pulleys disposed at end portions, a conveyance belt attached to the two pulleys, and a motor, but is not limited thereto.

In FIG. 2, when the motor for the x-axis-direction actuator 16 rotates, the belt rotates in conjunction with the rotation of the motor via one of the pulleys, and the ultraviolet source 14 moves in the x-direction along with rotational movement of the belt. When the motor for the y-axis-direction actuator 17 rotates, the belt rotates in conjunction with the rotation of the motor via one of the pulleys, and the ultraviolet source 14 moves in the y-direction along with rotational movement of the belt. When the motor for the z-axis-direction actuator 18 rotates, the belt rotates in conjunction with the rotation of the motor via one of the pulleys, and the ultraviolet source 14 moves in the z-direction along with rotational movement of the belt.

By movement, the ultraviolet irradiation unit 13 can irradiate the stage 1 with the ultraviolet light 15 emitted from the ultraviolet source 14 while moving the ultraviolet light 15.

Here, it is desirable that the ultraviolet irradiation unit 13 prevents the ultraviolet light 15 from being emitted to a portion where the irradiation of the ultraviolet light 15 is desired to be avoided on the stage 1, for example, the unused consumables area 7 where a consumable made of a resin material is placed.

Therefore, the ultraviolet source 14 is moved in each of the three directions, that is, the x-direction, the y-direction, and the z-direction such that the ultraviolet light 15 can be emitted to the target position where no rubber material or the like is used among another sample installation part 2, the disposal box 3, the extraction part 10, the reaction part 5, the detection part 6, the reagent storage 9, and the like while avoiding the unused consumables area 7.

For this reason, the ultraviolet source 14 can be configured to be turned on when it is determined that all of an x coordinate, a y coordinate, and a z coordinate of the ultraviolet source 14 on the stage 1 are within a predetermined value range set to enable irradiation with the ultraviolet light 15 at the target position, and not allowed to be turned on when any one or more of the x coordinate, the y coordinate, and the z coordinate are not within the predetermined value range, but is not limited thereto and is not particularly limited.

For example, there is a method of moving only a predetermined coordinate as a method for preventing the ultraviolet light 15 from being emitted to the portion where the irradiation of the ultraviolet light 15 is desired to be avoided on the stage 1, for example, the unused consumables area 7 where the consumable made of the resin material is placed. This is implemented by moving the ultraviolet source 14 according to coordinates stored in the control part 8B. The coordinates include the x-direction, the y-direction, and the z-direction.

The irradiation of the ultraviolet light 15 by the ultraviolet irradiation unit 13 is executed according to an instruction of a user on an operation screen displayed on the monitor 8A of the operating part 8, and may also be executed in various forms such as automatically executing the irradiation when a predetermined time elapses or executing the irradiation when power of the analysis device 30 is turned on or off.

The ultraviolet light 15 can irradiate the entire target position, and alternatively may irradiate only a part of the target position.

It is desirable that movement between irradiation target areas of the ultraviolet light 15 of the ultraviolet source 14 can be performed along a shortest distance in the x-direction, the y-direction, and the z-direction.

Next, effects of present embodiment will be described.

The analysis device 30 of the first embodiment of the invention described above includes the detection part 6 that performs analysis of the sample, the sample installation part 2 that performs necessary processing in analysis performed by the detection part 6, the disposal box 3, the reaction part 5, the unused consumables area 7, the operating part 8, the reagent storage 9, the extraction part 10, and the stage 1 on which the detection part 6, the sample installation part 2, the disposal box 3, the reaction part 5, the unused consumables area 7, the operating part 8, the reagent storage 9, and the extraction part 10 are disposed and which constitutes a portion of the housing of the analysis device 30, and further includes the ultraviolet source 14 that emits the ultraviolet light to the target position on the stage 1, and the x-axis-direction actuator 16, the y-axis-direction actuator 17, and the z-axis-direction actuator 18 that move the ultraviolet source 14 in the x-direction that is one direction of the stage 1, the y-direction at a right angle to the x-direction, and the z-direction that is the vertical direction.

Accordingly, since it is possible to limit a range where the ultraviolet light 15 reaches, it is possible to limit an irradiation range such that an easily-contaminated portion is irradiated whereas a portion where resin, rubber, or the like is used is not irradiated with the ultraviolet light 15. Even when there is a height difference on the stage 1 having a height difference, the ultraviolet light 15 can be moved in the z-direction such that a large number of target positions can be irradiated with the ultraviolet light 15. Therefore, it is possible to prevent component deterioration due to the ultraviolet irradiation, and it is also possible to freely change irradiation intensity of the ultraviolet light 15 to be the same or different for each target position.

In the embodiment, a barcode reader 25, a distance sensor 26, a Peltier element 27, a heat sink 28, and a cooling fan 29 described in a second embodiment to be described later can be appropriately mounted.

### <Second Embodiment>

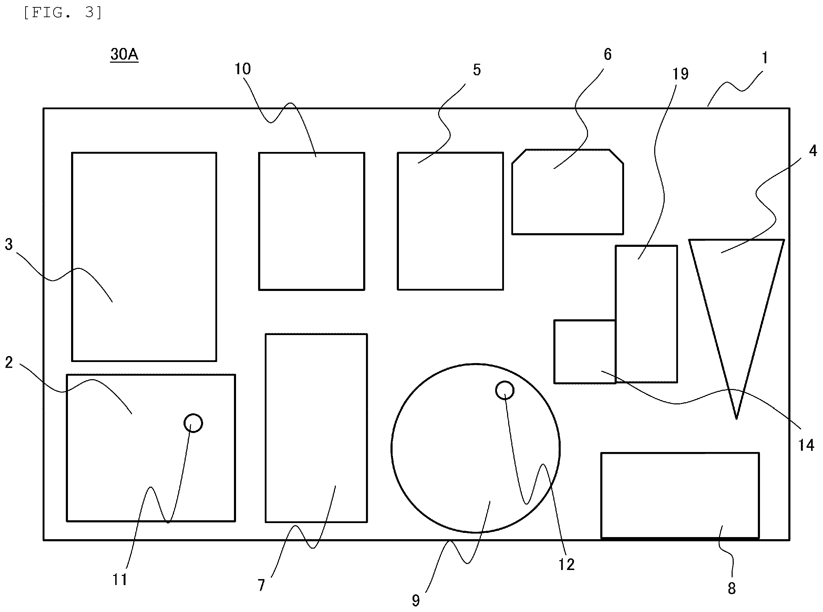

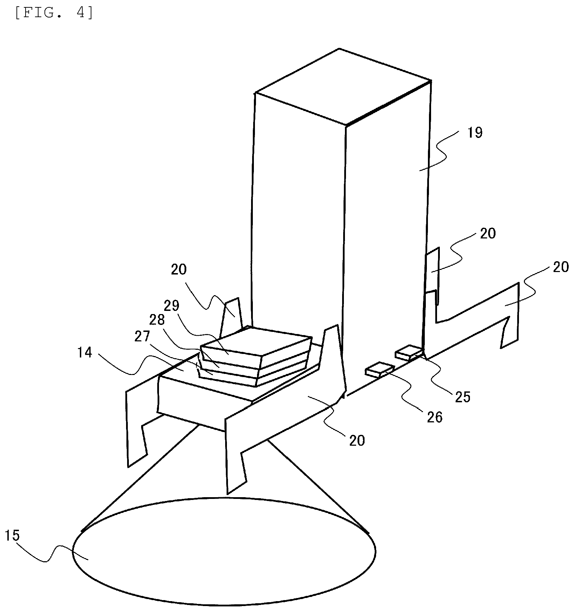

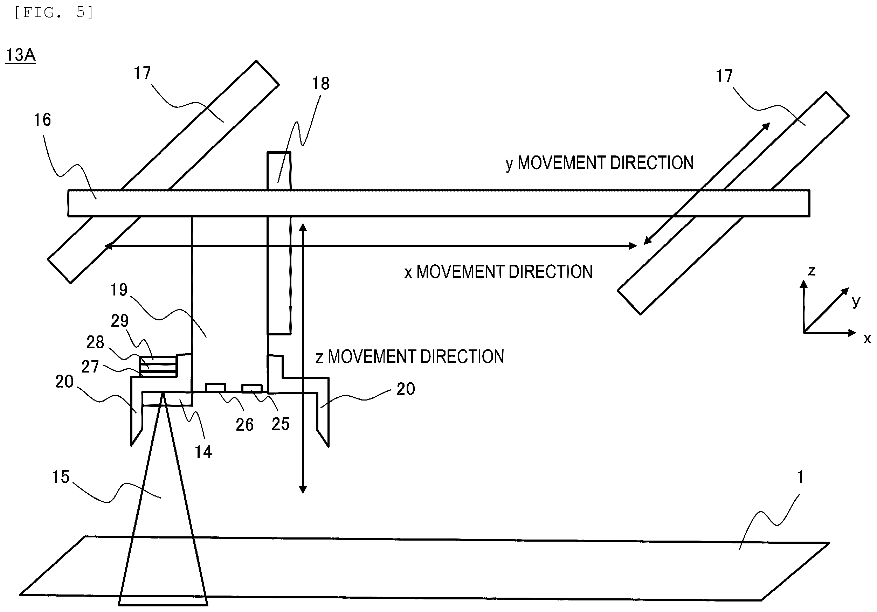

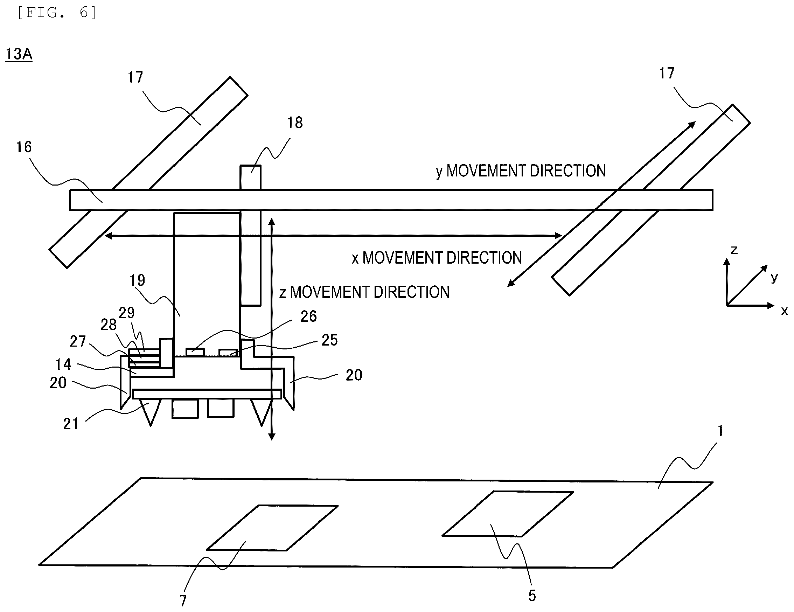

An analysis device and an ultraviolet irradiation unit according to a second embodiment of the invention will be described with reference to FIGS. 3 to 6. FIG. 3 is a diagram showing a schematic configuration of the analysis device according to the second embodiment, FIG. 4 is a diagram showing a schematic configuration around an ultraviolet source in the ultraviolet irradiation unit, and FIGS. 5 and 6 are diagrams showing an overview of a moving method for the ultraviolet irradiation unit.

An analysis device 30A of the embodiment shown in FIG. 3 further includes a gripper mechanism 19 in addition to the components of the analysis device 30 of the first embodiment shown in FIG. 1. Specifically, the gripper mechanism 19 including grippers 20 is mounted on the x-axis-direction actuator 16, the y-axis-direction actuator 17, and the z-axis-direction actuator 18 of an ultraviolet irradiation unit 13A, and the ultraviolet source 14 is mounted on the gripper mechanism 19.

As shown in FIG. 4, the grippers 20 are jigs that grip articles used in the detection part 6, the sample installation part 2, the disposal box 3, the reaction part 5, the unused consumables area 7, the operating part 8, the reagent storage 9, and the extraction part 10, and move the articles used in the detection part 6, the sample installation part 2, the disposal box 3, the reaction part 5, the unused consumables area 7, the operating part 8, the reagent storage 9, and the extraction part 10 to predetermined positions by being moved in x-y-z directions by the x-axis-direction actuator 16, the y-axis-direction actuator 17, and the z-axis-direction actuator 18.

The ultraviolet source 14 is interposed between the grippers 20, but the ultraviolet source 14 may not necessarily be provided between the grippers 20.

In addition to the grippers 20, the barcode reader 25, the distance sensor 26, the Peltier element 27, the heat sink 28, and the cooling fan 29 are mounted on the gripper mechanism 19.

The distance sensor 26 is a device that measures a distance to the target position, and measures, for example, a distance from the ultraviolet source 14 to the target position. A measurement result by the distance sensor 26 is received by the operating part 8, and only when the distance is within a predetermined range, the ultraviolet source 14 is turned on to emit ultraviolet light.

The barcode reader 25 is a machine that reads a barcode attached to the sample container containing the sample placed on the sample installation part 2 or a barcode attached to a reagent container containing the reagent stored in the reagent storage 9.

The Peltier element 27 is a device for cooling the ultraviolet source 14, a heat absorption side is in close contact with the ultraviolet source 14, and a heat dissipation side is in close contact with the heat sink 28. The Peltier element 27 may be always powered on, or may be turned on when increasing ultraviolet light intensity.

The heat sink 28 is made of a material having high thermal conductivity such as copper and is a jig for dissipating heat generated by the ultraviolet source 14.

The cooling fan 29 is a fan provided on the heat sink 28 and is a component for cooling the ultraviolet source 14. Similarly to the Peltier element 27, the cooling fan 29 may be always turned on, or may be turned on when increasing the ultraviolet light intensity. On and off of the Peltier element 27 and the cooling fan 29 are not necessarily synchronized, and can be freely set.

By providing any one or more of the Peltier element 27, the heat sink 28, and the cooling fan 29, an amount of current supplied to the ultraviolet source 14 can be increased to increase light quantity, and an effect of shortening an irradiation time can be obtained.

As shown in FIG. 5, since the gripper mechanism 19 is mounted on the x-axis-direction actuator 16, the y-axis-direction actuator 17, and the z-axis-direction actuator 18, the gripper mechanism 19 is configured to be movable in the three-dimensional x-y-z directions in the analysis device 30A. When the gripper mechanism 19 moves, the ultraviolet source 14 also moves in the three-dimensional x-y-z directions in the device, and the stage 1 can be irradiated with the ultraviolet light 15 emitted from the ultraviolet source 14 while moving the ultraviolet light 15.

Therefore, for example, as shown in FIG. 6, the grippers 20 can grip a consumable 21 provided in the unused consumables area 7 on the stage 1, and the gripper mechanism 19 can move onto the reaction part 5 to place the consumable 21.

The ultraviolet source 14 can irradiate the stage 1 with the ultraviolet light 15 while avoiding the portion where the irradiation with the ultraviolet light 15 is desired to be avoided, for example, the unused consumables area 7.

The ultraviolet light 15 may not be emitted when the grippers 20 grip the consumable 21.

Other configurations and operations are substantially the same as configurations and operations of the analysis device and the ultraviolet irradiation unit of the first embodiment described above, and details thereof will be omitted.

In the analysis device and the ultraviolet irradiation unit of the second embodiment of the invention, substantially the same effects as those of the analysis device and the ultraviolet irradiation unit of the first embodiment described above can still be obtained.

Since the gripper mechanism 19 that moves the articles used in the detection part 6, the sample installation part 2, the disposal box 3, the reaction part 5, the unused consumables area 7, the operating part 8, the reagent storage 9, and the extraction part 10 is further provided, and the ultraviolet source 14 is mounted on the gripper mechanism 19, movement axes of the gripper mechanism 19 originally provided in the analysis device can be used, and thus it is not necessary to prepare axes for the ultraviolet source 14. Therefore, since it is not necessary to provide three new actuators for the ultraviolet source 14, a device configuration can be simplified and a weight can be reduced.

Further, by providing the distance sensor 26 that measures the distance to the target position, it is also possible to determine whether the distance irradiated with the ultraviolet light 15 is within a predetermined range, and it is possible to further reduce a possibility that the ultraviolet light 15 is emitted to the outside of a desired range.

### <Third Embodiment>

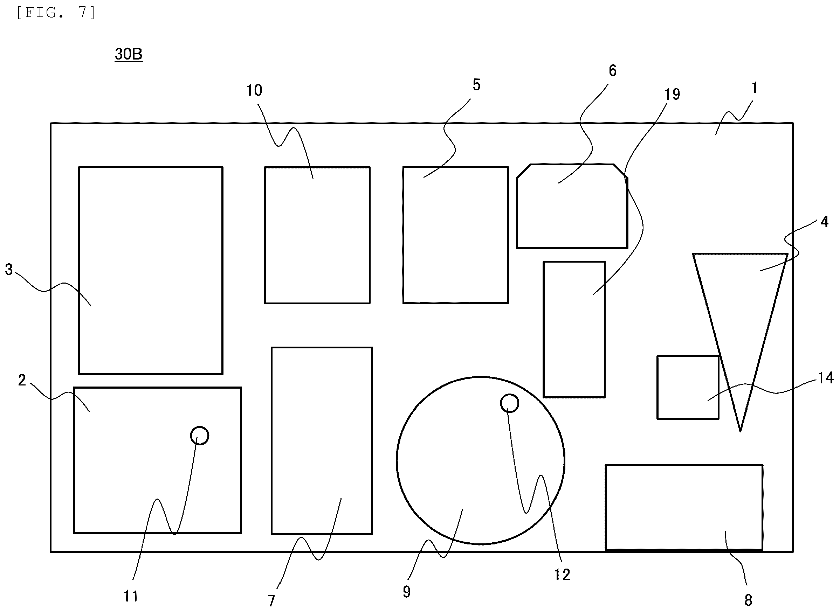

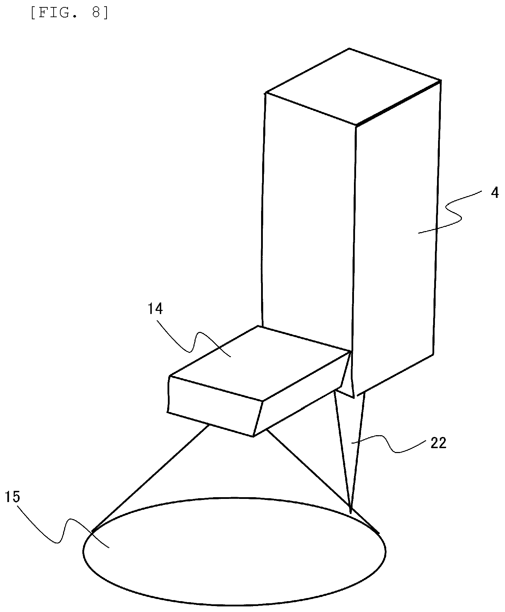

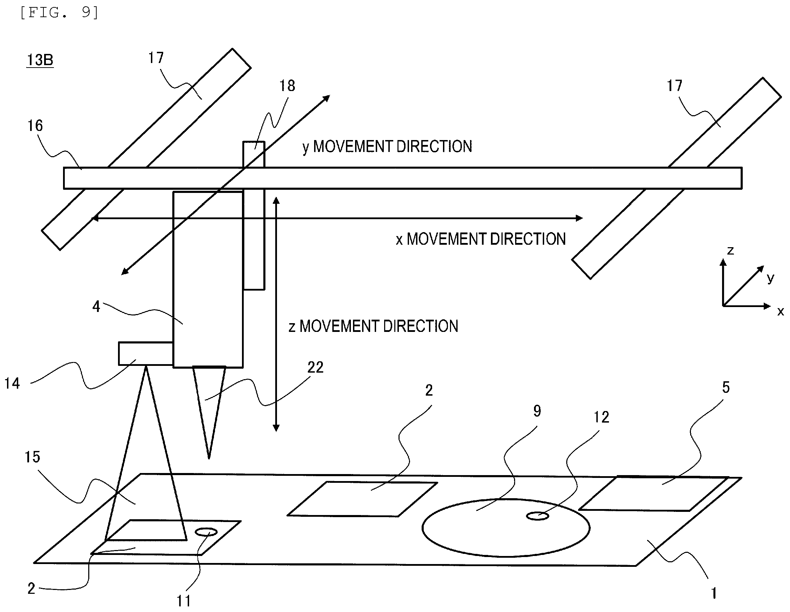

An analysis device and an ultraviolet irradiation unit according to a third embodiment of the invention will be described with reference to FIGS. 7 to 9. FIG. 7 is a diagram showing a schematic configuration of the analysis device according to the third embodiment, FIG. 8 is a diagram showing a schematic configuration of the ultraviolet irradiation unit, and FIG. 9 is a diagram showing an overview of a moving method for the ultraviolet irradiation unit.

In an analysis device 30B of the embodiment shown in FIG. 7, the ultraviolet source 14 is mounted on the dispenser 4 of the analysis device 30 of the first embodiment shown in FIG. 1. That is, it can be said that the dispenser 4 is provided as a part of an ultraviolet irradiation unit 13B.

As shown in FIG. 8, the ultraviolet source 14 is attached to the dispenser 4. The ultraviolet source 14 is connected to the dispenser 4. The ultraviolet source 14 emits the ultraviolet light 15.

FIG. 9 is a schematic diagram of an operation of the dispenser 4. The dispenser 4 is connected to the x-axis-direction actuator 16, the y-axis-direction actuator 17, and the z-axis-direction actuator 18, and the dispenser 4 can move in the analysis device 30B. When the dispenser 4 moves, the ultraviolet source 14 also moves.

The dispenser 4 can be moved in the device by the x-axis-direction actuator 16, the y-axis-direction actuator 17, and the z-axis-direction actuator 18. For example, the sample is dispensed to a dispensing tip 22 from the sample dispensing hole 11 of the sample installation part 2 on the stage 1, and then the sample is moved to the extraction part 10 and discharged. Similarly, the reagent from the reagent dispensing hole 12 of the reagent storage 9 is dispensed from the reagent dispensing hole 12 to the dispensing tip 22, then the reagent is moved to the extraction part 10 and discharged to a corresponding portion of the reaction part 5 to which the sample has been previously discharged.

The ultraviolet source 14 can irradiate the stage 1 with the ultraviolet light 15 while avoiding the portion where the irradiation with the ultraviolet light 15 is desired to be avoided, for example, the unused consumables area 7.

The ultraviolet light 15 may not be emitted when the dispenser 4 performs a dispensing operation.

Other configurations and operations are substantially the same as configurations and operations of the analysis device and the ultraviolet irradiation unit of the first embodiment described above, and details thereof will be omitted.

Since the analysis device and the ultraviolet irradiation unit according to the third embodiment of the invention further include the dispenser 4 that dispenses the sample, and the ultraviolet source 14 is mounted on the dispenser 4, it is still possible to obtain substantially the same effects as those of the analysis device and the ultraviolet irradiation unit according to the second embodiment described above, that is, it is not necessary to newly prepare an actuator for the ultraviolet source 14, and thus a device configuration can be simplified and a weight can be reduced.

The gripper mechanism 19 and the dispenser 4 may be integrated. The dispenser 4 can also be appropriately equipped with any one or more of the distance sensor 26, the Peltier element 27, the heat sink 28, and the cooling fan 29 described in the second embodiment.

### <Fourth Embodiment>

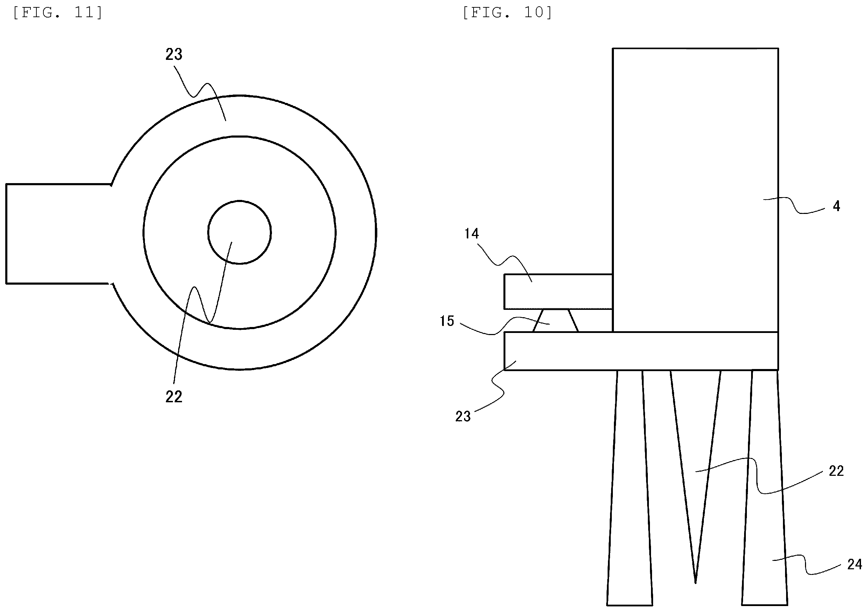

An analysis device and an ultraviolet irradiation unit according to a fourth embodiment of the invention will be described with reference to FIGS. 10 and 11. FIG. 10 is a diagram showing a schematic configuration of the ultraviolet irradiation unit according to the fourth embodiment, and FIG. 11 is a top view showing a positional relationship between a light guide and a dispensing tip in the ultraviolet irradiation unit.

The analysis device of the embodiment shown in FIGS. 10 and 11 further includes a ring-shaped light guide 23 disposed around the ultraviolet source 14 attached to the dispenser 4 in the analysis device of the third embodiment.

As shown in FIGS. 10 and 11, the ultraviolet light 15 emitted from the ultraviolet source 14 enters the light guide 23. The light guide 23 emits ring-shaped ultraviolet light 24 to surround the dispensing tip 22.

As shown in FIG. 11, when the dispenser 4 performs dispensing, the ring-shaped ultraviolet light 24 emitted from the light guide 23 is emitted. Since the ring-shaped ultraviolet light 24 is emitted to surround the dispensing tip 22 during dispensing, contamination by bacteria and viruses during dispensing can be prevented.

Other configurations and operations are substantially the same as configurations and operations of the analysis device and the ultraviolet irradiation unit of the third embodiment described above, and details thereof will be omitted.

In the analysis device and the ultraviolet irradiation unit of the fourth embodiment of the invention, substantially the same effects as those of the analysis device and the ultraviolet irradiation unit of the third embodiment described above can still be obtained.

Since the light guide 23 disposed around the ultraviolet source 14 is further provided, the stage is irradiated with ultraviolet light to surround the dispensing tip 22 and the like, and thus it is possible to sterilize and inactivate bacteria and viruses susceptible to ultraviolet light. Accordingly, it is possible to prevent contamination by bacteria and viruses during dispensing.

### <Fifth Embodiment>

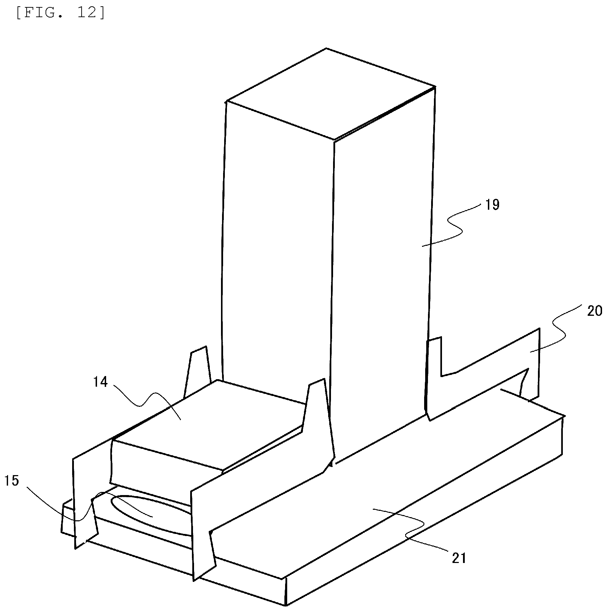

An analysis device and an ultraviolet irradiation unit according to a fifth embodiment of the invention will be described with reference to FIG. 12. FIG. 12 is a diagram showing a schematic configuration of the ultraviolet irradiation unit according to the fifth embodiment.

In the analysis device of the embodiment shown in FIG. 12, the grippers 20 are controlled to emit ultraviolet light while gripping and moving the article.

As shown in FIG. 12, when the grippers 20 of the gripper mechanism 19 grip the consumable 21, the ultraviolet source 14 irradiates the consumable 21 with the ultraviolet light 15 and moves the consumable 21 to a next destination. At this time, the consumable 21 at a portion irradiated with the ultraviolet light 15 can obtain sterilization and inactivation effects before being moved to the reaction part 5, for example.

For example, in the case of the consumable 21 after the reaction in the reaction part 5 is completed, since bacteria and viruses adhering to the consumable 21 are sterilized and inactivated by irradiation with the ultraviolet light 15 when the consumable 21 is moved to the disposal box 3, it is possible to reduce a risk of infection of an operator due to bacteria and viruses in the disposal box where the operator performs collection. In addition, since the ultraviolet light is applied while moving, a time of a workflow can be shortened.

In this embodiment, since the ultraviolet source 14 is provided such that the ultraviolet light 15 is emitted only to the consumable, it is possible to prevent the ultraviolet light 15 from being emitted to the portion where irradiation is desired to be prevented on the stage 1.

Other configurations and operations are substantially the same as configurations and operations of the analysis device and the ultraviolet irradiation unit of the second embodiment described above, and details thereof will be omitted.

In the analysis device and the ultraviolet irradiation unit of the fifth embodiment of the invention, substantially the same effects as those of the analysis device and the ultraviolet irradiation unit of the second embodiment described above can still be obtained.

Since the irradiation with the ultraviolet light is performed when the grippers 20 grip and move the article, it is not necessary to stop the irradiation, and thus an ultraviolet irradiation time can be shortened.

### <Sixth Embodiment>

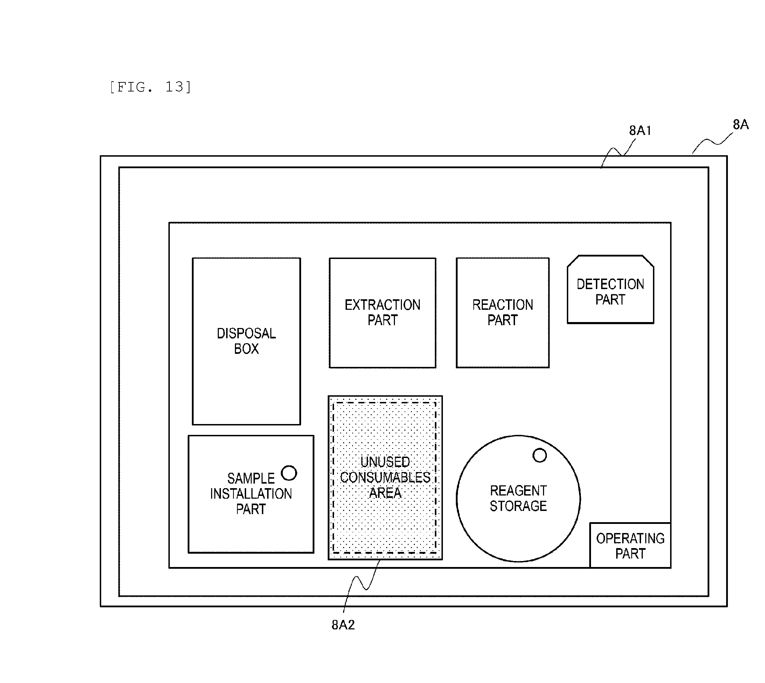

An analysis device and an ultraviolet irradiation unit according to a sixth embodiment of the invention will be described with reference to FIG. 13. FIG. 13 is a diagram showing an example of display of an operating part in the analysis device according to the sixth embodiment.

In the analysis device of the embodiment, an operation screen 8A1 for performing ultraviolet irradiation setting is displayed on the monitor 8A of the operating part 8, and the operator of the analysis device can freely set an irradiation time, an irradiation position, and the like.

On the operation screen 8A1 on which a portion where irradiation with the ultraviolet light 15 is desired can be selected from the operating part 8 of the analysis device shown in FIG. 13, it is possible to select the portion where irradiation with the ultraviolet light 15 is desired on the screen, for example, the sample dispensing hole 11 of the sample installation part 2. After area selection, the irradiation time can also be input, and the irradiation with the ultraviolet light 15 can be performed.

As shown in FIG. 13, on the operation screen 8A1 displayed on the monitor 8A of the operating part 8, for example, a mask area 8A2 is set for the unused consumables area 7, and an area where ultraviolet irradiation is to be avoided can be clearly indicated.

The control part 8B of the operating part 8 moves the x-axis-direction actuator 16, the y-axis-direction actuator 17, and the z-axis-direction actuator 18 such that the ultraviolet light 15 can be emitted to the portion set by the operation screen 8A1.

The ultraviolet source 14 mounted on the x-axis-direction actuator 16, the y-axis-direction actuator 17, and the z-axis-direction actuator 18 may be mounted on the ultraviolet irradiation unit 13 as in the first embodiment, may be mounted on the gripper mechanism 19 as in the second embodiment, or may be mounted on the dispenser 4 as in the third embodiment, which is not particularly limited.

Other configurations and operations are substantially the same as configurations and operations of the analysis device and the ultraviolet irradiation unit of the first to fifth embodiments described above, and details thereof will be omitted.

In the analysis device and the ultraviolet irradiation unit of the sixth embodiment of the invention, substantially the same effects as those of the analysis device and the ultraviolet irradiation unit of the first to fifth embodiments described above can still be obtained.

By further providing the operation screen 8A1 for performing the ultraviolet irradiation setting, a degree of freedom in ultraviolet irradiation setting is improved, and it is possible to perform ultraviolet irradiation according to a circumstance where the analysis device is placed.

### <Others>

The invention is not limited to the above-described embodiments, and includes various modifications. The embodiments described above have been described in detail to describe the invention in an easy-to-understand manner, and the invention is not necessarily limited to those including all configurations described above.

A part of a configuration according to a certain embodiment can also be replaced with a configuration according to another embodiment, and a configuration according to another embodiment can be added to a configuration according to a certain embodiment. In addition, another configuration can be added to a part of a configuration of each embodiment, and a part of a configuration of each embodiment can be deleted or replaced with another configuration.

For example, although the mechanism for moving in a "Cartesian coordinate system" represented by the x-axis-direction actuator 16, the y-axis-direction actuator 17, and the z-axis-direction actuator 18 has been described as an example of the conveyance mechanism for moving the ultraviolet source 14 in the x-direction that is one direction of the stage 1, the y-direction at a right angle to the x-direction, and the z-direction that is the vertical direction, a mechanism for moving the ultraviolet source in axial directions of a "cylindrical coordinate system" represented by a radius (R) from a center, an angle (θ) in a radial direction, and a distance (Z) in a cylindrical axis direction may be appropriately adjusted to move in the x-direction, the y-direction at a right angle to the x-direction, and the z-direction that is the vertical direction.

### Reference Signs List

- 1: stage
- 2: sample installation part (processing part)
- 3: disposal box (processing part)
- 4: dispenser
- 5: reaction part (processing part)
- 6: detection part (analysis part)
- 7: unused consumables area (processing part)
- 8: operating part (processing part)
- 8A: monitor
- 8A1: operation screen (setting part)
- 8A2: mask area
- 8B: control part
- 9: reagent storage (processing part)
- 10: extraction part (processing part)
- 11: sample dispensing hole
- 12: reagent dispensing hole
- 13, 13A, 13B: ultraviolet irradiation unit
- 14: ultraviolet source
- 15: ultraviolet light
- 16: x-axis-direction actuator (conveyance mechanism)
- 17: y-axis-direction actuator (conveyance mechanism)
- 18: z-axis-direction actuator (conveyance mechanism)
- 19: gripper mechanism
- 20: gripper
- 21: consumable
- 22: dispensing tip
- 23: light guide
- 24: ring-shaped ultraviolet light
- 25: barcode reader
- 26: distance sensor
- 27: Peltier element
- 28: heat sink
- 29: cooling fan
- 30, 30A, 30B: analysis device

## Claims

1. An analysis device comprising:
an analysis part configured to perform analysis of a sample;
a processing part configured to perform necessary processing in analysis performed by the analysis part;
a stage on which the analysis part and the processing part are disposed and which constitutes a portion of a device housing;
an ultraviolet source configured to emit ultraviolet light to a target position on the stage; and
a conveyance mechanism configured to move the ultraviolet source in an x-direction that is one direction of the stage, a y-direction at a right angle to the x-direction, and a z-direction that is a vertical direction.

2. The analysis device according to claim 1, further comprising:
a gripper mechanism configured to move an article used in the analysis part or the processing part, wherein
the ultraviolet source is mounted on the gripper mechanism.

3. The analysis device according to claim 1, further comprising:
a distance sensor configured to measure a distance to the target position.

4. The analysis device according to claim 1, further comprising:
a dispenser configured to dispense the sample, wherein
the ultraviolet source is mounted on the dispenser.

5. The analysis device according to claim 4, further comprising:
a light guide disposed around the ultraviolet source.

6. The analysis device according to claim 2, wherein
the ultraviolet light is emitted when the gripper mechanism moves while gripping the article.

7. The analysis device according to claim 1, further comprising:
a setting part configured to perform irradiation setting of the ultraviolet light.

8. An ultraviolet irradiation unit comprising:
an ultraviolet source configured to emit ultraviolet light; and
a conveyance mechanism configured to move the ultraviolet source in an x-direction that is one direction of a housing to which the conveyance mechanism is to be mounted, a y-direction at a right angle to the x-direction, and a z-direction that is a vertical direction, wherein
the ultraviolet light is emitted to a target position on the housing.
